(19) Europäisches Patentamt
European Patent Office
Office européen des brevets

(11) Publication number: **0 103 636**
**B1**

(12) # EUROPEAN PATENT SPECIFICATION

(45) Date of publication of patent specification: **12.09.90**

(51) Int. Cl.[5]: **A 61 K 9/22,** A 61 K 31/485

(21) Application number: **83901458.6**

(22) Date of filing: **14.03.83**

(86) International application number:
**PCT/US83/00333**

(87) International publication number:
**WO 83/03197 29.09.83 Gazette 83/23**

(54) **USE OF OPIUM ANTAGONISTS FOR THE MANUFACTURE OF MEDICAMENTS FOR CONTROLLING GASTROINTESTINAL DYSMOTILITY.**

(30) Priority: **16.03.82 US 358820**
**04.02.83 US 464110**

(43) Date of publication of application:
**28.03.84 Bulletin 84/13**

(45) Publication of the grant of the patent:
**12.09.90 Bulletin 90/37**

(84) Designated Contracting States:
**AT BE CH DE FR GB LI LU NL SE**

(56) References cited:
**DE-A-2 822 882**
**GB-A-1 522 480**
**US-A-3 060 086**
**US-A-3 214 341**
**US-A-3 254 088**
**US-A-3 320 262**
**US-A-3 332 950**
**US-A-3 344 029**
**US-A-3 896 226**
**US-A-4 176 186**
**US-A-4 272 541**
**US-A-4 277 605**

**The file contains technical information submitted after the application was filed and not included in this specification**

(73) Proprietor: **ROCKEFELLER UNIVERSITY**
**1230 York Avenue**
**New York, NY 10021 (US)**

(72) Inventor: **KREEK, Mary Jeanne**
**1161 York Avenue Apartment 12L**
**New York, NY 10021 (US)**
Inventor: **FISHMAN, Jack**
**867 Park Avenue Apartment 5N**
**New York, NY 10021 (US)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

**EP 0 103 636 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Gastrointestinal dysmotility affects many humans and is associated with various clinical signs and syndromes. Hypomotility is associated with chronic constipation, obstipation, idiopathic abdominal distention, abdominal pain, abdominal cramps, irritable bowel syndrome, non-tropical sprue, megacolon associated with hypothyroidism, pseudo-obstruction of the gastrointestinal tract, colitis, hypomotility of the colon associated with diabetes mellitus, adult onset Hirschsprung's disease, neorological disorders, myopathic disorders, geriatric hypomotility disorders, jejunal-ileal bypass with secondary magacolon, hypomotility associated with cancer chemotherapy, hypomotility associated with severe burns and other major stresses, hypomotility associated with syndromes of depression, post-operative intestinal distension, and other pathological conditions. Gastrointestinal hypomotility disorders also include other disorders of esophageal and gastric-motility and gastric emptying disorders such as diabetic gastric paresis, scleroderma and other disorders. Idiopathic constipation is a major health problem which affects many individuals. Millions of persons utilize laxatives, stool softeners, fiber preparations, mineral oil, gas absorbants, suppositories or enemas on a continuous basis. Partial hypomotility is a major feature of several defined gastrointestinal disorders.

Hypomotility is often associated with recurring bouts of hypermotility, the so-called intermittent hypomotility-hypermotility syndrome. Clinical manifestations of this affliction include alternate bouts of constipation and diarrhea, abdominal distention, pains and cramps, ileitis, regional enteritis, generalized irritable bowel syndrome, irritable colon syndrome, ulcerative and other forms of colitis.

Opioid antagonists are a well recognized class of chemical agents. They have been described in detail in the scientific and patent literature.

Pure opioid antagonists are agents which specifically reverse the effects of opioid agonists, bind to specific opioid receptors but have no opioid agonist activity.

This invention is concerned with the use of pure opioid antagonists in contrast to opioid agonists and agents as defined below that manifest mixed agonist-antagonist activities such as pentazocine and buprenorphine.

US—A—4176186 discloses that certain quaternary noroxymorphones prevent or relieve the intestinal mobility inhibiting side-effects of narcotic analgesics without interfering with the analgesic activity of the latter.

In US—A—4277605 there are described 3-hydroxy-7-oxomorphinons and 3-hydroxy-7-oxoisomorphinons which are reported to be orally active antidiarrheal agents.

It has now been discovered that human disorders related to gastrointestinal dysmotility in humans can be improved, thereby alleviating the above noted illnesses, by administration of therapeutically effective amounts of pure opioid antagonists falling within the scope of formula I, II and III such as naloxone, naltrexone or nalmefene.

Accordingly, the present invention is directed to the use of compounds of the general formulae given below and the pharmaceutically acceptable salts thereof for the manufacture of medicaments for restoring intestinal dysmotility which is independent of prior administration of opioid antagonists.

The pharmaceutically active product will normally be administered orally or parenterally. In some cases both routes may be employed either sequentially or simultaneously. The dosage regimen which has been found to be most effective is 2 to 70 mg per day. The preferred dosage for oral administration is 10 to 50 mg per day, and for parenteral administration 10 to 70 mg per day. For sustained release forms of the medicament, either oral or parenteral dosage forms are useful which deliver 10 to 50 mg per day. Of course, sustained release forms can be prepared which will deliver proportional amounts over selected periods of time, for example 4, 6 or 12 hours. These quantities, irrespective of the method or route of administration selected, appear to provide optimum relief for adults in the 60 to 70 kg weight class. The attending physician may choose to vary the defined quantities depending on such factors as the condition being treated, and the age, weight, and general physical condition of the patient.

The principal and preferred compounds which are used in this invention are naloxone, naltrexone and nalmefene. These compounds are known narcotic antagonists. They are generally recognized as pure opioid antagonists and will be so regarded for purposes of this description. Naltrexone, however, has been described as having slight agonistic activity. Wikler, A., *Int. J. of the Addictions* 12(7) 869, 1977.

It should be noted, and is here emphasized, that the opioid antagonists as used in this invention are not used to neutralize the effect of opioid agonists such as narcotic drugs. They are used to treat clinical gastroenterologic disorders in which intestinal dysmotility is a major component. For such use they may be employed to treat intermittent or prolonged periods of hypomotility or intermittent hypomotility-hypermotility.

It has now been discovered that hypomotility arises from relative or absolute excess of one or more of the endogenous opioids at the intestinal level, in the brain or at both sites, or from abnormal binding of those endogenous opioids to their specific receptors in the intestine and/or brain, thereby causing inhibition of propulsive intestinal contractions. The use of pure opioid antagonists in accordance with this invention restores normal endogenous opioid balance and alleviates dysmotility problems.

Some humans, such as those suffering from chronic constipation, are affected with chronic hypomotility. Some individuals may suffer from hypermotility at one time and hypomotility another. As

indicated above, the effect of the therapeutic agents used in this invention when used as described herein is to restore the balance between available and bound opiate receptor sites thereby to restore normal motility as evidenced by relief of constipation, relief of abdominal distention or pain as illustrated in the examples.

Naloxone, naltrexone and nalmefene are representatives of known classes of compounds which are pure opioid antagonists. The compounds of the class are derivatives of morphine and codeine.

Nalmefene is typical of one useful class of compounds which are described together with their method of preparation in United States Patent US—A—3,896,226 which was issued on July 22, 1975. The compounds are morphine or codeine derivatives which may be represented by the formula:

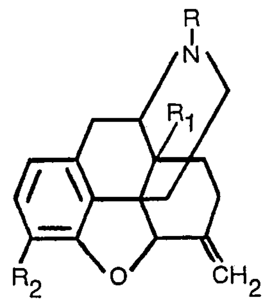

wherein R is allyl or cyclopropylmethyl, $R_1$ is hydrogen or hydroxy and $R_2$ is hydroxy or methoxy.

Typical compounds within the scope of the formula include:

a. 6-methylene-6-desoxy-N-allyl-14-hydroxydihydronormorphine.
b. 6-methylene-6-desoxy-N-cyclopropylmethyl-14-hydroxydihydronormorphine.
c. 6-methylene-6-desoxy-N-cyclopropylmethyl dihydronormorphine.
d. 6-methylene-6-desoxy-N-allyl-dihydronormorphine.
e. 6-methylene-6-desoxy-N-allyl-dihydronorcodeine.
f. 6-methylene-6-desoxy-N-cyclopropylmethyl-14-hydroxydihydronorcodeine.
g. 6-methylene-6-desoxy-N-allyl-14-hydroxydihydronorcodeine.

Compound *b* is nalmefene.

The compounds are prepared by reaction of the appropriate 6-keto starting compound with excess triphenylphosphomethylene followed by reaction with an allyl or cyclopropylmethyl halide, suitably the bromide or chloride. If the starting compound is a 3-hydroxy compound, i.e., a morphine derivative, the final product can be converted to a codeine derivative by reaction with diazomethane to convert the hydroxyl group to a methoxyl moiety.

Other morphine derivatives which may be employed in the practice of the invention as well as methods for their preparation are described in United States Patents US—A—3,254,088 and 3,332,950. They may be represented by the formula:

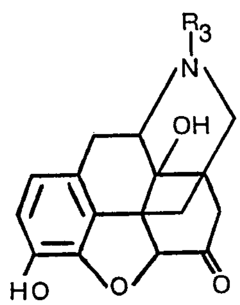

wherein $R_3$ is allyl, 3-methyl-2-butenyl, cyclopropylmethyl or cyclobutylmethyl.

The compounds may be prepared by reaction of a selected 14-hydroxy starting compound with a suitable organic halide such as 1-bromo-3-methyl-2-butene, allyl bromide or the corresponding chlorides.

Typical compounds within the scope of the foregoing formula include:

a. N-allyl-14-hydroxydihydronormorphinone.
b. N-cyclopropylmethyl-14-hydroxydihydronormorphinone.
c. N-cyclobutylmethyl-14-hydroxydihydronormorphinone.
d. N-(3-methyl-2-butenyl)-14-hydroxydihydronormorphinone.

The first named compound is naloxone. The second is naltrexone.

A third class of pure antagonists useful in this invention is described along with methods of preparation in United States Patent US—A—3,320,262. The compounds are represented by the formulas shown below in which the second formula represents dihydro compounds:

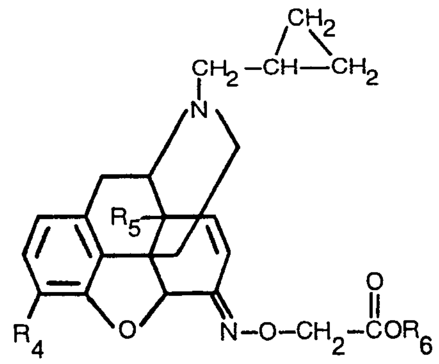

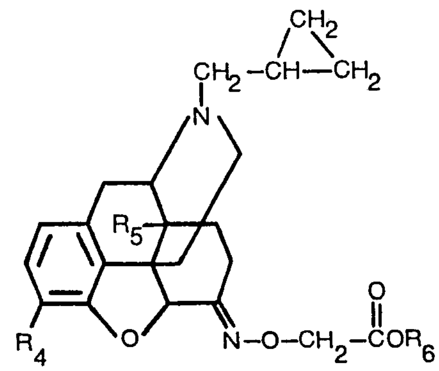

wherein $R_4$ is methoxy or hydroxy, $R_5$ is hydrogen or hydroxy and $R_6$ is hydrogen, methyl, ethyl, propyl, allyl or benzyl.

Typical compounds within the scope of the formula include:

a. N-cyclopropylmethyl-nor-14-hydroxycodeinone-6-carboxymethyloxime.
b. N-cyclopropylmethyl-nor-codeinone-6-carboxymethyloxime.
c. N-cyclopropylmethyl-nor-14-hydroxymorphinone-6-carboxymethyloxime.
d. N-cyclopropylmethyl-nor-morphinone-6-carboxymethyloxime methylester.
e. N-cyclopropylmethyl-nor-14-hydroxydihydrocodeinone-6-carboxy-methyloxime.
f. N-cyclopropylmethyl-nor-14-hydroxydihydrocodeinone-6-carboxy-methyloxime methylester.
g. N-cyclopropylmethyl-nor-dihydrocodeinone-6-carboxy-methyloxime methylester.
h. N-cyclopropylmethyl-nor-14-hydroxydihydromorphinone-6-carboxy-methyloxime methylester.
i. N-cyclopropylmethyl-nor-dihydromorphinone-6-carboxy-methyloxime methylester.

The compounds are prepared by reaction of the selected ketone starting compound with a suitable organic halide as described above, followed by reaction of the N-substituted compounds with a selected ester of carboxymethoxyl amine.

All of the compounds described above can be utilized in the form of pharmaceutically acceptable salts. Pharmaceutically acceptable salts are salts which are free of toxicity or other therapeutically harmful or undesirable effects. These include, for example, such salts as the hydrochloride, hydrobromide, neutral and acid fumarate and maleate, terephthalate, ethane sulfonate, oxalate and bitartrate.

Water-soluble salts with volatile acids (e.g. hydrochloric and acetic acid) can be prepared by adding an aqueous solution of slightly more than one equivalent of the acid to an aqueous dispersion of the base and evaporating the solution thus formed under reduced pressure. The residue can then be recrystallized. Salts of non-volatile inorganic acids (e.g. orthophosphoric acid) can be prepared by adding the stoichiometric amount of the acid to an aqueous dispersion of the base and treating the resulting solution in the same way. Salts of organic acids which are difficultly soluble in water (e.g. the benzoate) can be prepared by reacting the acid and the base in equivalent amounts in ethyl alcohol medium and evaporating the solution.

For ease of administration it is, of course, preferred to treat patients orally. Surprisingly, as is illustrated hereinafter, naloxone, which has been art recognized as being poorly absorbed when given perorally is nevertheless active at local intestinal sites and effective for the indications mentioned above when administered orally. In the event that the patient is unable to cooperate, is kept in a "nothing by mouth" status, if there is an intransigent blockage of the gastrointestinal tract, or if antagonist activity at the brain or at other systemic sites is desired, the route of choice will be the parenteral route.

The principal aspects of the invention, then, are the use of certain known compounds for the manufacture of medicaments for controlling intestinal dysmotility. The compounds may be used alone or in association with selected pharmaceutical carriers in the form of pharmaceutical compositions containing effective amounts of the active agents.

The compositions may be administered parenterally or by various non-parenteral routes, primarily oral, but also by buccal, sublingual, rectal and transdermal routes. The compositions may be prepared for relatively rapid absorption or in sustained release forms.

For buccal and sublingual administration the active ingredient can be formulated in tablet form with water soluble binding agents such as lactose or other palatable carbohydrates.

For rectal administration suppositories or inserts containing the active ingredient dispersed in such reagents as cocoa butter, petroleum, or other natural lubricant or in a synthetic emollient such as polyethylene glycol 1000 or polyethylene glycol 4000, may be manufactured.

Transdermal administration will normally be from a sustained release preparation which may be applied as a patch or from a gauze applied to the skin.

The preferred method of administering the active agents of this invention is from sustained release forms since this is most convenient for patients, and avoids the necessity of constant clock watching or interruption of normal daily activities. A number of compositions suitable for such preparations are known and can be used in the practice of this invention. As aforesaid, the dosage forms can be prepared to deliver 10 to 50 mg of active ingredient per day divided over selected time intervals, for example 4, 6, 12 or even twenty-four hours.

One convenient procedure is to formulate the selected motility control agent in a time disintegrating tablet or pellet coated with various thicknesses of known materials such as carnauba wax, cellulose esters and ethers, fats, keratin, gluten or various natural or synthetic esters. Tablets in which the motility control agent is contained in a slowly dissolving core such as a core of stearic acid or castor oil are useful. Mixed release granule tablets comprising mixtures of the drug itself and the drug in separate particles coated with materials which dissolve at different rates such as dehydrogenated castor oil or fatty acids can also be employed. Alternatively the active material can be bound to an ion exchange resin such as sulfuric acid type cation exchange resin.

The presently preferred sustained release forms of this invention are those in which the active agent is carried through the gastrointestinal tract in a mixed polymer carrier. The carrier slowly erodes during transport so that increments of the opioid antagonist may be released for attachment to receptor sites.

In these forms, the principal carrier is a mixed, hydrated alkyl hydroxy cellulose in which the alkyl groups contain up to four carbon atoms and at least one is propyl or butyl. This polymer functions as a drug release retardant. Cellulose derivatives which are substituted with two different alkyl groups are preferred since there is less tendency for such polymers to crystallize. The polymers are prepared by standard alkoxylation reactions. In case two different alkyl groups are to be substituted, concurrent or successive reactions may be employed. Generally, with such mixed substituents there will be about 50% of each substituent. The presently preferred polymer is propyl hydroxymethyl cellulose.

The cellulose derivative is normally hydrated to a degree of from about 5% to 25% by weight, preferably 10% to 20%. A degree of hydration of 15% by weight is especially preferred since it is readily achieved, and provides sustained release forms with excellent properties.

To prevent the cellulose polymer from crystallizing and thereby reducing the rate at which the active agent is released, an anticrystallinity agent is added. The function of the agent is to prevent the cellulose polymer from achieving a degree of regularity at which it will crystallize. The presently preferred anticrystallinity reagents are polyalkylene oxides, such as polyethylene oxide or other pharmaceutically acceptable analogues. The molecular weight of the oxide may vary from about 100,000 to 10 million with 4 to 5 million being preferred because of ready availability, ease of compounding and efficiency for preventing crystallization. Normally the amount of such agent added will be from about 15% to 30% by weight so that the weight of cellulose product will be from about 70% to 85%.

The polymer and selected active agent or agents are compounded, to form tablets or other standard dosage forms in conventional equipment with any of a number of anti-stick or releasing agents such as magnesium stearate or talc. The amount employed is not critical and normally ranges from about 0.5% to 2% by weight.

The sustained release dosage forms can be formulated to contain any desired quantity of active agent. Typically, a tablet or other form will contain from 5% to 20% active ingredient and 80% to 95% carrier. As indicated above, they may be prepared to release the selected quantities of opioid antagonist over time periods of, for example, from 4 to 12 hours.

In the foregoing discussions, all quantities given on a by weight basis are based on the total weight except the degree of hydration of the cellulose derivative which is based on the weight of the derivative.

In short, the motility control agents used according to this invention can be administered in any of a wide variety of forms including tablets, capsules, lozenges, suppositories, emulsions and isotonic solutions. They can be formulated for immediate absorption or for sustained release.

The following examples are given by way of illustration only.

Example I

A female with a history of chronic constipation for a period of over twenty years, requiring daily use of large doses of laxatives plus frequent enemas, was admitted to the research hospital on day one at approximately 4:00 p.m. She was given a standard laxative regimen documented to be inadequate to effect a spontaneous bowel movement, and placed on a high residue diet.

During the first 24 hours she was treated with a placebo (Infusion A) according to the following schedule:

| | | |
|---|---|---|
| 4 p.m.—5 p.m. | 1) Dextrose and water | 250 ml over 1 hr. |
| 5 p.m.—12 midnight | 2) Saline | 250 ml over 7 hrs. |
| 12 midnight—8 a.m. | 3) Dextrose and water | 250 ml over 8 hrs. |
| 8 a.m.—4 p.m. | 4) Dextrose and water | 250 ml over 8 hrs. |
| | | 1250 ml over 24 hrs. |

On day two at 4:00 p.m. the placebo was terminated and the patient was treated intravenously with naloxone according to the following schedule.

| | | |
|---|---|---|
| 4 p.m.—5 p.m. | 1) Dextrose & Water | 250 ml + 1.6 mg Naloxone in 1 hr. |
| 5 p.m.—12 midnight | 2) Saline | 250 ml + 8.4 mg Naloxone in 7 hrs. |
| 12 midnight—8 a.m. | 3) Dextrose & Water | 250 ml + 9.6 mg Naloxone in 8 hrs. |
| 8 a.m.—4 p.m. | 4) Dextrose & Water | 500 ml + 9.6 mg Naloxone in 8 hrs. |
| | | 1250 ml + 29.2 mg Naloxone in 24 hrs. |

At 4:00 p.m. on day three the parenteral administration was discontinued and the dosage regimen was changed to oral administration of 3.6 mg of naloxone in synthetic grape juice every three hours for three days, omitting treatment only when the patient was sleeping. The total dosage per day was 21.6 mg.

During the five days that the patient was under hospital care the stools were collected and weighed. The results were as follows:

| | Stool Weight Grams | |
|---|---|---|
| Day | Wet Weight | Dry Weight |
| 1 | 452 | 41 |
| 2 | 649 | 52 |
| 3 | 985 | 77 |
| 4 | 997 | 77 |
| 5 | 806 | 65 |

Samples of the patient's blood and urine were collected each day and subjected to analysis. No adverse effects were observed.

The experiment was conducted as a single blind. The patient was not aware of whether she was receiving a placebo or the active agent.

Example II

A second patient with a history of over twenty years of chronic constipation, intractable to laxatives, who for the past several months was only relieved by enema treatment was admitted to the hospital and placed on a high residue diet.

She was then treated in a manner similar to the patient of Example I, but with no laxatives or enemas given, in accordance with the following schedule:

| Day | Treatment |
|---|---|
| 1 | 29.2 mg naloxone, intravenous |
| 2 | placebo, oral |
| 3 | 14.4 mg naloxone, oral |
| 4 | 21.6 mg naloxone, oral |
| 5 | 21.6 mg naloxone, oral |
| 6 | 7.2 mg naloxone, oral |
| 7 | placebo, oral |
| 8 | placebo, oral |
| 9 | placebo, oral |

The patient's stools were collected and weighed each day with the following results:

| | STOOL WEIGHT GRAMS | |
|---|---|---|
| Day | Wet Weight | Dry Weight |
| 1 | 84 | 18.5 |
| 2 | none | - |
| 3 | 35 | 8.2 |
| 4 | 125 | 66.4 |
| 5 | 230 | 82.4 |
| 6 | 34 | 12.1 |
| 7 | none | - |
| 8 | 43 | 12.2 |
| 9 | none | - |

No adverse effects were noted by clinical examinations, blood or urine analysis.

The following tables summarize the results which were achieved with additional patients treated with naloxone following the general procedures of Examples I and II. Substantially the same results will be achieved with naltrexone, nalmefene and other compounds within the scope of the foregoing formulas.

The tables set forth the significant details and results of a number of studies conducted with different patients suffering from various forms of intestinal dysmotility. For example, Table 1 reports the results of five separate studies, three inpatient and two outpatient, on the same patient at the research hospital. The first study was of two days duration. The lengths of subsequent studies are as indicated.

The reports Tables 1 to 5 are for patients with chronic constipation. Those in Tables 6, 7 and 8 are for patients with irritable bowel syndrome. Tables 9 and 10 are summaries.

TABLE 1

Patient Number 1
A.O., a 53 year old female

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 (inpat-ient) | i.v. | 1 | P | – | 452 | 41 | 1.8 | No change |
| | | 2 | A | 29.2 | 649 | 52 | 3.0 | Marked improvement |
| #2 (inpat-ient) | p.o. | 1 | A | 21.6 | 985 | 77 | 4.7 | Marked improvement |
| | | 2 | A | 21.6 | 997 | 77 | 3.3 | Marked improvement |
| | | 3 | A | 21.6 | 806 | 65 | 2.4 | Marked improvement |
| #3 (outpat-ient) | p.o. | 1 | A | 21.6 | | | | Average day |
| | | 2 | A | 21.6 | | | | Better than average day |
| | | 3 | A | 21.6 | | | | Much better than average day |
| | | 4 | P | – | | | | Average day |
| | | 5 | P | – | | | | Bad day |
| | | 6 | P | – | | | | Bad day |

TABLE 1 (continued)

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #4 (inpat-ient) | i.v. | 1 | neither | - | 336 | 15 | 2.6 | |
| | | 2 | A | 29.2 | 775 | 199 | 5.7 | |
| | | 3 | A | 29.2 | 859 | 212 | 4.1 | |
| #5 (outpat-ient) | | 1 | A | 21.6 | | | | Much better than average |
| | | 2 | A | 21.6 | | | | Much better than average |
| | | 3 | A | 21.6 | | | | Much better than average |
| | | 4 | P | - | | | | Average (diarrhea) |
| | | 5 | P | - | | | | Worse than average |

TABLE 2

Patient Number 2
B.K., a 30 year old female

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 | – | 1 | neither | 0 | | | | |
| | | 2 | A | 29.2 | 84 | 18.5 | 3.6 | |
| | | 3 | P | 0 | – | – | – | No stool |
| #2 | – | 1 | A | 14.4 | 35 | 8 | 1.1 | |
| | | 2 | A | 21.6 | 125 | 66 | 5.9 | |
| | | 3 | A | 21.6 | 230 | 82 | 9.0 | |
| | | 4 | A | 7.2 | 34 | 12 | 0.8 | |
| | | 5 | P | 0 | – | – | – | No stool |
| | | 6 | P | 0 | 43 | 7 | 0.9 | |
| | | 7 | P | 0 | – | – | – | No stool |
| | | 8 | P | 0 | 30 | 7 | 0.8 | |
| | | 9 | – | 0 | 37 | 10 | 0.9 | |

TABLE 3

Patient Number 3
J.P., a 39 year old male

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 | i.v. | 1 | A | 29.2 | - | - | - | No stool |
| | | 2 | P | - | - | - | - | No stool |
| | | 3 | - | - | - | - | - | No stool |
| | | 4 | - | - | - | - | - | No stool |
| | | 5 | - | - | - | - | - | No stool |
| #2 | p.o. | 1 | - | - | - | - | - | No stool |
| | | 2 | - | - | - | - | - | No stool |
| | | 3 | - | - | - | - | - | No stool |
| | | 4 | - | - | - | - | - | No stool |
| | | 5 | A | 21.6 | - | - | - | No stool |

TABLE 3 (continued)

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| | | 6 | A | 21.6 | - | - | - | No stool |
| | | 7 | A | 21.6 | - | - | - | No stool |
| | | 8 | P | - | - | - | - | No stool |
| | | 9 | P | - | - | - | - | No stool |
| | | 10 | P | - | - | - | - | No stool |

TABLE 4

Patient Number 7
B.B., a 65 year old female

| Study | Route of Administration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 (in-patient) | i.v. | 1 | A | 29.2 | 166 | 59 | - | Cramps; improvement Spontaneous passage of stool |
| | | 2 | P | - | 205 | 42 | - | Average; digital removal of stool |
| #2 (in-patient) | p.o. | 1 | A | 21.6 | - | missing | - | No stool |
| | | 2 | A | 21.6 | 238 | 35 | - | Improvement;"natural easy BM's" |
| | | 3 | A | 21.6 | 262 | 35 | - | Marked improvement;spontaneous "natural BM's" |
| | | 4 | A | 21.6 | 145 | 20 | - | Marked improvement;spontaneous "natural BM's" |
| | | 5 | P | - | 265 | 23 | - | Marked improvement;spontaneous "natural BM's" |
| | | 6 | P | - | 299 | 36 | - | Marked improvement;spontaneous "natural BM's" |
| | | 7 | P | - | 166 | 38 | - | Less improvement;less BM;still spontaneous |

TABLE 4 (continued)

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| | | 8 | P | - | 105 | 11 | - | BM only with digital removal; back to average |
| | | 9 | - | - | - | - | - | No BM even after attempt at digital removal |
| #3 (in-patient) | i.v. | 1 | P | - | - | - | - | |
| | | 2 | P | - | 135 | 17 | - | Removal by digital manipulation |
| | | 3 | A | 29.2 | - | - | - | |
| | | 4 | A | 29.2 | 183 | 24 | - | Spontaneous BM; some of feces lost because of sudden urge to pass BM |

TABLE 5

Patient Number 8
H.D., a 71 year old male

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 | i.v. | 1 | P | - | 144 | 12 | - | |
| | | 2 | A | 29.2 | 281 | 17 | - | Marked improvement |
| #2 | p.o. | 1 | A | 21.6 | 135 | 7 | - | Marked improvement |
| | | 2 | A | 21.6 | 134 | 14 | - | Marked improvement |
| | | 3 | A | 21.6 | 476 | 29 | - | Marked improvement |
| | | 4 | P | - | - | - | - | Spontaneous BM; but feces lost by patient because of sudden urge to pass BM |
| | | 5 | P | - | 554 | 26 | - | Improvement |
| | | 6 | P | - | 114 | 9 | - | Strain to pass stool |

TABLE 6

Patient Number 4
B.S., a 68 year old male

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 (in-patient) | i.v. | 1 | - | - | 21 | 2.0 | 0.7 | Pain; usual type of bad day |
| | | 2 | A | 29.2 | 14 | 1.0 | 0.3 | Marked improvement; pain; much better than average |
| | | 3 | P | - | 43 | 4.0 | 6.4 | Sustained improvement; pain; much better than average |
| #2 (out-patient) | p.o. | 1 | A | 21.6 | | | | Marked improvement; no pain; full BM |
| | | 2 | A | 21.6 | | | | No records kept; grant-child's death |
| | | Grandchild died; postponed trial for 2 days | | | | | | |
| | | 3 | A | 21.6 | | | | Marked improvement; full BM; no pain |
| | | 4 | P | - | | | | Marked improvement; no pain; considerable gas; full BM |
| | | 5 | P | - | | | | Some improvement; no pain; considerable gas |

TABLE 6 (continued)

| Study | Route of Adminis- tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| | | 6 | P | – | | | | Average day; abdominal distension; gas; difficulty passing BM |
| #3 (out-patient) | p.o. | 1 | P | – | | | | Pain; distension; poor day |
| | | 2 | P | – | | | | Problem with passing BM; pain; average day |
| | | 3 | P | – | | | | Pain; problem passing BM; average day |
| | | 4 | P | – | | | | Pain; problem passing BM; average day |
| | | 5 | P | – | | | | Pain; problem passing BM; average day |
| | | 6 | A | 14.4 | | | | Pain; problem passing BM; average day |
| | | 7 | A | 21.6 | | | | Good BM; no pain; marked improvement |
| | | 8 | A | 21.6 | | | | Good BM; followed later by pain; some improvement |

TABLE 6 (continued)

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| | | 9 | A | 21.6 | | | | Good BM; no pain; marked improvement |
| | | 10 | A | 10.8 | | | | Good BM; no pain; marked improvement |
| | | 11 | A | 18.0 | | | | Good BM; some pain; some gas |
| #4 (out-patient) | p.o. | 1 | A | 7.2 | | | | Pain; distention; average day |
| | | 2 | A | 18.0 | | | | Pain; distention; average day |
| | | 3 | A | 21.6 | | | | Pain; spontaneous BM; slight improvement |
| | | 4 | A | 18.0 | | | | Pain; distention; some improvement |
| | | 5 | A | 21.6 | | | | No pain; minimal distention; marked improvement |
| | | 6 | P | – | | | | No pain; some distention; some improvement |
| | | 7 | P | – | | | | Distention; recurrent pain |

TABLE 6 (continued)

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| | | 8 | P | - | | | | Abdominal pain; some distention; slight improvement |
| | | 9 | P | - | | | | Distention; no pain; slight improvement |

TABLE 7

Patient Number 5
J.B., a 19 year old female

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 (in-patient) | i.v. | 1 | A | 29.2 | 61 | 14 | 2.6 | Less distention; better than average day |
| | | 2 | P | - | 648 | 204 | 5.7 | Less distention; better than average day |
| #2 (out-patient) | p.o. | 1 | A | 10.8 | 253 | 75 | 0.8 | Less distention |
| | | 2 | A | 21.6 | | | | Marked improvement; spontaneous BM |
| | | 3 | A | 21.6 | | | | Spontaneous BM; some distention |
| | | 4 | P | - | | | | Distention; pain; cramps; marked deterioration |
| | | 5 | P | - | | | | Spontaneous BM; some pain; distention |
| | | 6 | P | - | | | | Distention; spontaneous BM |
| #3 (in-patient) | i.v. | 1 | P | - | 12 | 1. | | No change |
| | | 2 | A | 29.2 | 96 | 31 | | Much less distention; spontaneous passage of multiple soft BM |

TABLE 7 (continued)

| Study | Route of Adminis-tration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #4 (out-patient) | p.o. | 1 | A | 21.6 | | | | 3 BM passed; no pain; no distention |
| | | 2 | A | 21.6 | | | | 3 BM soft passed; no pain, no distention |
| | | 3 | A | 21.6 | | | | 2 BM soft passed; some cramps; no distention |
| | | 4 | P | - | | | | Small hard BM passed; distention |
| | | 5 | P | - | | | | 2 small BM; pain; distention |
| | | 6 | P | - | | | | 2 small BM; pain; distention |

TABLE 8

Patient Number 6
A.S., a 66 year old male

| Study | Route of Administration | Study Day | Active Compound or Placebo | Total 24 Hour Dose (mg) | Fecal Wet Wt. (gm) | Fecal Dry Wt. (gm) | Fecal Fat (%) | Symptoms, Signs, Comments |
|---|---|---|---|---|---|---|---|---|
| #1 (in-patient) | i.v. | 1 | A | 29.2 | - | - | - | No BM; marked improvement with decreased pain and distention |
| | | 2 | P | - | 21 | 4 | 1.6 | BM small; decreased pain and distention |
| #2 (out-patient) | | 1 | A | 18.0 | 60 | 14 | 2.1 | Spontaneous BM; no distention; marked improvement |
| | | 2 | A | 21.6 | 44 | 6 | 2.3 | Spontaneous BM; no distention; marked improvement |
| | | 3 | A | 21.6 | | | | No BM; no pain or distention |
| | | 4 | A | 21.6 | | | | Spontaneous BM; no symptoms |
| | | 5 | A | 21.6 | | | | No stool; no symptoms |
| | | 6 | A/P | 3.6 | | | | Spontaneous BM; no symptoms |
| | | 7 | P | - | | | | No stool; average day |
| | | 8 | P | - | | | | One BM; better than average day |
| | | 9 | P | - | | | | No stool; average day |

TABLE 9

Chronic Constipation Patients

| Patient Initials | Patient Number | Age | Sex | Diagnosis | Onset of Symptoms | No. of Inpatient Studies | No of Out-patient Studies (all P.O.) | Outcome |
|---|---|---|---|---|---|---|---|---|
| A.O. | 1 | 53 | F | Non-tropical sprue | more than 40 years | 3 (2 iv; 1 po) | 2 | 5 successful trials |
| B.K. | 2 | 39 | F | Idiopathic con-stipation | more than 20 years | 2 (1 iv; 1 po) | 0 | 2 successful trials |
| J.P. | 3 | 39 | M | Idiopathic con-stipation | about 10 years | 2 (1 iv; 1 po) | 0 | No successful trials |
| B.B. | 7 | 65 | F | Idiopathic con-stipation | more than 50 years | 3 (2 iv; 1 po) | 1 | 2 successful trials; 1 probably successful trial |
| H.D. | 8 | 77 | M | Geriatric type idiopathic constipation | about 8 years | 2 (1 iv; 1 po) | 0 | 1 successfultrial; 1 probably successful trial |

TABLE 10

Irritable Bowel Syndrome Patients

| Patient Initials | Patient Number | Age | Sex | Onset of Symptoms | Number of Inpatient Studies | Number of Outpatient Studies (all P.O.) | Outcome |
|---|---|---|---|---|---|---|---|
| B.S. | 4 | 68 | M | More than 40 years | 1 i.v. | 3 | Successful |
| J.B. | 5 | 19 | F | More than 10 years | 2 i.v. | 2 | Successful |
| A.S. | 6 | 66 | M | About 5 years | 1 i.v. | 1 | Successful |

The following formulations illustrate procedures which can be employed to produce a variety of dosage forms of the active ingredients used according to this invention. The active ingredient in each formulation is naloxone. It could be naltrexone, nalmefene or any of the other compounds used according to this invention.

TABLET

| | | mg Tablet |
|---|---|---|
| 1) Oral form: | Naloxone hydrochloride | 12 |
| | Starch | 50 |
| | Lactose | 75 |
| | Magnesium stearate | 2 |
| | Stearic acid | 5 |

The compound, a portion of the starch and the lactose are combined and wet granulated with starch paste. The wet granulation is placed on trays and allowed to dry overnight at a temperature of 45°C. The dried granulation is comminuted in a comminutor to a particle size of approximately 20 mesh. Magnesium stearate, stearic acid, and the balance of the starch are added and the entire mix blended prior to compression on a suitable tablet press. The tablets are compressed at a weight of 232 mg using a 2.3 cm (11/12″) punch with a balance of 4 kg. These tablets will disintegrate within a half hour according to the method described in USP XVI.

| | | mg Tablet |
|---|---|---|
| 2) | Naloxone | 6 |
| | Microcrystalline cellulose | 30 |
| | Spray-dried lactose | 60 |
| | Colloidal silica | 1 |
| | Stearic acid | 1 |

Screen the naloxone to break up lumps and blend with microcrystalline cellulose. Add spray-dried lactose and blend. Finally add the stearic acid and colloidal silica; blend to obtain homogenous mixture. Compress using 0.7 cm (9/32 in) shallow concave punch.

CAPSULES

| | | |
|---|---|---|
| 1) | Naloxone hydrochloride | 10 mg |
| | Lactose | 45 |
| | Starch | 45 |

The compound, a portion of the starch and the lactose are combined and wet granulated with starch paste. The wet granulation is placed on trays and allowed to dry overnight at a temperature of 45°C. The dried granulation is added to a hand gelatin capsule of the appropriate size.

| | | |
|---|---|---|
| 2) | Naloxone | 20 mg |
| | Sesame oil | 90 |

The free base is mixed with sesame oil and encapsulated in a soft gelatin capsule of the appropriate size.

SUSTAINED RELEASE

1) Oral

Naloxone (12 mg) is included in a hydrophilic polymer matrix from which it will be gradually excluded following ingestion. The inclusion is accomplished by dissolving the free base in a suitable non-polar solvent from which it will be absorbed by the polymers. Removal of the solvent leaves the product bound in the matrix from which it is released by water solution. Rates of delivery can be controlled by the hydrophilic character of the matrix which can be both non- or biodegradable as desired.

2) Oral

Naloxone (12 mg) is mixed with sucrose and compounded into 1 mm diameter pellets to yield a total of 200 pellets. Fifty beads are used in the uncoated form. The remaining beads are divided into three equal parts and coated with stearic acid, palmitic acid and glycerol myristate in appropriate amounts to allow dissolution in the intestine over 4, 8 or 12 hours. The beads are encapsulated in an appropriate size hard gelatin capsule.

PARENTERAL

| | | mg/ml |
|---|---|---|
| 1) | Naloxone hydrochloride | 10 |
| | Methyl paraben | 1.8 |
| | Propyl paraben | 0.2 |
| | Water for injection | q.s. |

The solution is prepared by first dissolving the parabens in hot water for injection, cooling to room temperature and dissolving the compound and sodium chloride. It is then filtered, using sterile technique, through a bacteriological filter (0.6 µm or smaller porosity), after which it is transferred into ampules or multiple-dose vials.

| | | mg/ml |
|---|---|---|
| 2) | Naloxone | 10 or 20 |
| | Ethanol | 100 |
| | Propylene-glycol | 880 |

The solution is prepared by dissolving the naloxone in the alcohol and diluting with propylene glycol. It is then filtered using sterile techniques, through a bacteriological filter after which it is transferred into ampules or multiple dose vials.

SUSTAINED RELEASE

1) Parenteral

The hydrochloride salt of naloxone (20 mg) is dissolved in an appropriate amount of ethanol. The solution is mixed with sesame oil (5:1 ratio) and heated at 45°C under vacuum to remove the alcohol. The residue (drug in sesame oil) is transferred into individual or repeated dose ampules.

| 2) Suppository | Percent |
|---|---|
| Naloxone hydrochloride | 4.06 |
| Polyoxyethylene 1000 (approx M 1000) | 80.14 |
| Polyoxyethylene 4000 (approx M 4000) | 15.00 |
| Methyl paraben | 0.45 |
| Propyl paraben | 0.05 |
| Purified water USP | 3.10 |

The HCl salt of the compound is dissolved in the water and added to a melted mixture of the polyoxyethylenes which are already combined with the parabens. This molten mixture is poured into suppository molds and cast into suppositories weighing 3 grams each. They are frozen to solidify and packaged into foil.

3) Tablet
A total of 65 gm 15% hyrated propyl hydroxymethyl cellulose and 24 gm polyethylene oxide (mw = 5,000,000) are mixed together with 10 gm of naloxone hydrochloride in a uniform powdery slurry and 1 mg magnesium stearate is added. The mix is pressed into tablets at a pressure of 9.8 MPa (100 atm). Each of the tablets contains 10 mg naloxone. A tablet when orally administered delivers the naloxone over a period of 12 hours.

4) Tablet
Nalmefene tablets which are suitable for a sustained release delivery over 12 hour periods are prepared by mixing in the conventional manner 63 gm 15% hydrated propyl hydroxymethyl cellulose and 24 gm polyethylene oxide (mw = 5,000,000) together with 12 gm of nalmefene hydrochloride in a uniform powdery slurry. The mix is pressed into tablets at a pressure of 9.8 MPa (100 atm), each of the tablets containing 12 mg nalmefene.

## Claims

1. Use of a compound represented by the general formula I:

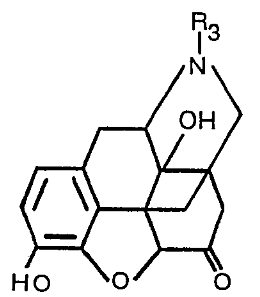

(I)

wherein $R_3$ is 3-methyl-2-butenyl, allyl, cyclopropylmethyl or cyclobutylmethyl;
and the pharmaceutically acceptable salts thereof for the manufacture of medicaments for restoring intestinal motility in a patient afflicted with an intestinal dysmotility which is independent of prior administration of opioid agonists.

2. Use according to claim 1 wherein the medicament is manufactured in a form suitable for oral administration.

3. Use according to claim 1 wherein the medicament is manufactured in a form suitable for parenteral administration.

4. Use according to claim 1 wherein the medicament is manufactured in the form of a sustained release formulation.

5. Use according to claim 4 wherein said sustained release formulation is manufactured to contain, based on the total weight, from 5 to 20% of compound(s) of formula I, including pharmaceutically acceptable salts thereof, and from 80 to 95% of a carrier, said carrier comprising a release agent together with from, based on the total weight, 70% to 30% of a hydrated alkyl hydroxy cellulose in which the alkyl groups contain up to four carbon atoms and at least one of them is propyl or butyl, and, based on the total weight, from 30% to 70% of a pharmaceutically acceptable polyalkylene oxide, the degree of hydration of the alkyl hydroxy cellulose being from 5% to 25% by weight, based on its total weight.

6. Use according to any one of claims 1 to 5 wherein the compound of formula I is naloxone or naltrexone.

7. Use according to any one of claims 5 and 6 wherein the hydrated alkyl hydroxy cellulose is propyl hydroxymethyl cellulose.

8. Use according to any one of claims 1 to 3 and 6 wherein the medicament is intended for the treatment of irritable bowel syndrome and/or constipation.

9. Use of a compound represented by the general formula II:

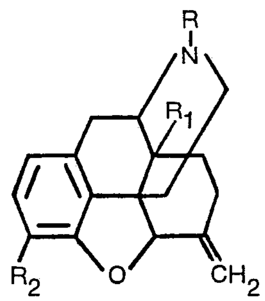

(II)

wherein
R is allyl or cyclopropylmethyl;
$R_1$ is hydrogen or hydroxy;
$R_2$ is hydroxy or methoxy;
and the pharmaceutically acceptable salts thereof for the manufacture of medicaments for restoring intestinal motility in a patient afflicted with an intestinal dysmotility which is independent of prior administration of opioid agonists.

10. Use according to claim 9 wherein the medicament is manufactured in a form suitable for oral administration.

11. Use according to claim 9 wherein the medicament is manufactured in the form of a sustained release formulation.

12. Use according to claim 11 wherein said sustained release formulation is manufactured to contain, based on the total weight, from 5 to 20% of compound(s) of Formula II, including pharmaceutically acceptable salts thereof, and from 80 to 95% of a carrier, said carrier comprising a release agent together with from, based on the total weight, 70% to 30% of a hydrated alkyl hydroxy cellulose in which the alkyl groups contain up to four carbon atoms and at least one of them is propyl or butyl, and, based on the total weight, from 30% to 70% of a pharmaceutically acceptable polyalkylene oxide, the degree of hydration of the alkyl hydroxy cellulose being from 5% to 25% by weight, based on its total weight.

13. Use according to any one of claims 9 to 12 wherein the compound of formula II is nalmefene.

14. Use according to any one of claims 9, 10 and 13 wherein the medicament is intended for the treatment of irritable bowel syndrome and/or constipation.

15. Use according to claim 12 wherein the hydrated alkyl hydroxy cellulose is propyl hydroxymethyl cellulose.

16. Use of a compound represented by the general formula III:

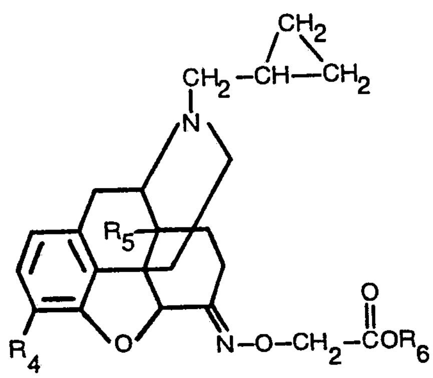

(III)

or the corresponding dehydro compound wherein:
$R_4$ is methoxy or hydroxy;
$R_5$ is hydrogen or hydroxy;
$R_6$ is hydrogen, methyl, ethyl, propyl, allyl or benzyl;
and the pharmaceutically acceptable salts thereof for the manufacture of medicaments for restoring intestinal motility in a patient afflicted with an intestinal dysmotility which is independent of prior administration of opioid agonists.

17. Use according to claim 16 wherein the medicament is manufactured in a form suitable for oral administration.

18. Use according to claim 16 wherein the medicament is manufactured in a form suitable for parenteral administration.

19. Use according to any one of claims 16 to 18 wherein the medicament is intended for the treatment of irritable bowel syndrome and/or constipation.

20. Use according to claim 16 wherein the medicament is manufactured in the form of a sustained release formulation.

21. Use according to claim 20 wherein the sustained release formulation is manufactured to contain, based on the total weight, from 5 to 20% of compound(s) of formula III, including corresponding dehydro compound(s) and pharmaceutically acceptable salts, and from 80 to 95% of a carrier, said carrier comprising a release agent together with from, based on the total weight, 70% to 30% of hydrated alkyl hydroxy cellulose in which the alkyl groups contain up to four carbon atoms and at least one of them is propyl or butyl, and, based on the total weight, from 30% to 70% of a pharmaceutically acceptable polyalkylene oxide, the degree of hydration of the alkyl hydroxy cellulose being from 5% to 25% by weight, based on its total weight.

22. Use according to claim 21 wherein the hydrated alkyl hydroxy cellulose is propyl hydroxymethyl cellulose.

## Patentansprüche

1. Verwendung einer Verbindung der allgemeinen Formel I:

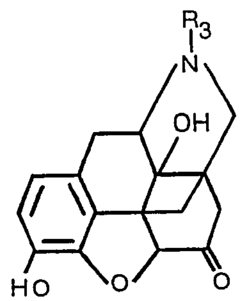

(I)

worin $R_3$ 3-Methyl-2-butenyl, Allyl, Cyclopropylmethyl oder Cyclobutylmethyl ist, und der pharmazeutisch annehmbaren Salze derselben zur Herstellung eines Arzneimittels, um die intestinale Motilität bei einem Patienten wiederherzustellen, der an intestinaler Dysmotilität leidet, die nicht von einer verherigen Verabreichung von Opioid-Agonisten abhängt.

2. Verwendung nach Anspruch 1, worin das Arzneimittel in einer zur oralen Verabreichung geeigneten Form hergestellt ist.

3. Verwendung nach Anspruch 1, worin das Arzneimittel in einer zur parenteralen Verabreichung geeigneten Form hergestellt ist.

4. Verwendung nach Anspruch 1, worin das Arzneimittel in Form einer Depot-Formulierung hergestellt ist.

5. Verwendung nach Anspruch 4, worin die Depot-Formulierung so hergestellt wird, daß sie, bezogen auf das Gesamtgewicht, 5 bis 20% Verbindung(en) der Formel I einschließlich pharmazeutisch annehmbarer Salze derselben und 80 bis 95% eines Trägers enthält, wobei der Träger ein Freisetzungsmittel zusammen mit (bezogen auf das Gesamtgewicht) 70 bis 30% einer hydratisierten Alkylhydroxycellulose, in welcher die Alkylgruppen bis zu 4 Kohlenstoffatome enthalten und mindestens eine der Alkylgruppen Propyl oder Butyl ist, und (bezogen auf das Gesamtegewicht) 30 bis 70% eines pharmazeutisch annehmbaren Polyalkylenoxids umfaßt, wobei der Hydrationsgrad der Alkylhydroxycellulose von 5 bis 25 Gew.-%, bezogen auf deren Gesamtgewicht, beträgt.

6. Verwendung nach irgendeinem der Ansprüche 1 bis 5, worin die Verbindung der Formel I Naloxon oder Natrexon ist.

7. Verwendung nach irgendeinem der Ansprüche 5 und 6, worin die hydratisierte Alkylhydroxycellulose Propylhydroxymethylcellulose ist.

8. Verwendung nach irgendeinem der Ansprüche 1 bis 3 und 6, worin das Arzneimittel zur Behandlung des irritablen Darmsyndroms und/oder der Konstipation beabsichtigt ist.

9. Verwendung einer Verbindung der allgemeinen Formel II:

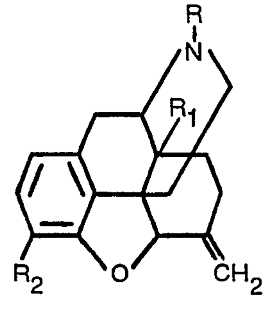

(II)

worin

R Allyl oder Cyclopropylmethyl ist;

$R_1$ Wasserstoff oder Hydroxy ist;

$R_2$ Hydroxy oder Methoxy ist;

und der pharmazeutisch annehmbaren Salze derselben zur Herstellung eines Arzneimittels, um die intestinale Motilität bei einem Patienten weiiederherzustellen, der an intestinaler Dysmotilität leidet, die nicht von der vorherigen Verabreichung von Opioid-Agonisten abhängt.

10. Verwendung nach Anspruch 9, worin das Arzneimittel in einer zur oralen Verabreichung geeigneten Form hergestellt ist.

11. Verwendung nach Anspruch 9, worin das Arzneimittel in Form einer Depot-Formulierung hergestellt ist.

12. Verwendung nach Anspruch 11, worin die Depot-Formulierung so hergestellt wird, daß sie, bezogen auf das Gesamtegewicht, 5 bis 20% Verbindung(en) der Formel II einschließlich pharmazeutisch annehmbarer Salze derselben und 80 bis 95% eines Trägers enthält, wobei der Träger ein Freisetzungsmittel zusammen mit (bezogen auf das Gesamtgewicht) 70 bis 30% einer hydratisierten Alkylhydroxylcellulose, in welcher die Alkylgruppen bis zu 4 Kohlenstoffatome enthalten und mindestens eine der Alkylgruppen Propyl oder Butyl ist, und (bezogen auf das Gesamtegewicht) 30 bis 70% eines pharmazeutisch annehmbaren Polyalkylenoxids umfaßt, wobei der Hydratationsgrad der Alkylhydroxycellulose 5 bis 25 Gew.-%, bezogen auf deren Gesamtgewicht, beträgt.

13. Verwendung nach irgendeinem der Ansprüche 9 bis 12, worin die Verbindung der Formel II Nalmefen ist.

14. Verwendung nach irgendeinem der Ansprüche 9, 10 und 13, worin das Arzneimittel zur Behandlung des irritablen Darmsyndrom und/oder der Konstipation beabsichtigt ist.

15. Verwendung nach Anspruch 12, worin die hydratisierte Alkylhydroxycellulose Propylhydroxymethylcellulose ist.

16. Verwendung einer Verbindung der allgemeinen Formel III

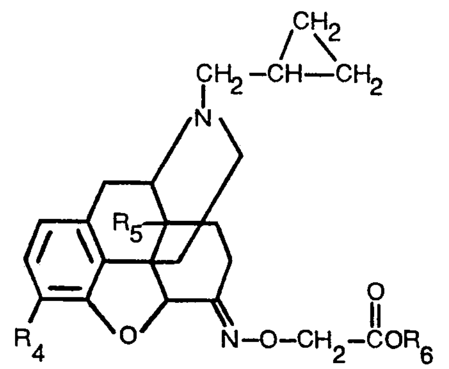

(III)

oder der entsprechenden Dehydroverbindung, worin

$R_4$ Methoxy oder Hydroxy ist;

$R_5$ Wasserstoff oder Hydroxy ist;

$R_6$ Wasserstoff, Methyl, Ethyl, Propyl, Allyl oder Benzyl ist;

und der pharmazeutisch annehmbaren Salze derselben zur Herstellung von Arzneimitteln, um die intestinale Motilität bei einem Patienten wiederherzustellen, der an intestinaler Dysmotilität leidet, die nicht von der verherigen Verabreichung von Opioid-Agonisten abhängt.

17. Verwendung nach Anspruch 16, worin das Arzneimittel in einer zur oralen Verabreichung geeigneten Form hergestellt ist.

18. Verwendung nach Anspruch 16, worin das Arzneimittel in einer zur parenteralen Verabreichung geeigneten Form hergestellt ist.

19. Verwendung nach irgendeinem der Ansprüche 16 bis 18, worin das Arzneimittel zur Behandlung des irritablen Darmsyndroms und/oder der Konstipation beabsichtigt ist.

20. Verwendung nach Anspruch 16, worin das Arzneimittel in Form einer Depot-Formulierung hergestellt ist.

21. Verwendung nach Anspruch 20, worin die Depot-Formulierung so hergestellt wird, daß sie, bezogen auf das Gesamtgewicht, 5 bis 20% Verbindung(en) der Formel III einschließlich der entsprechenden Dehydroverbindung(en) und entsprechender pharmazeutisch annehmbarer Salze und 80 bis 95% eines Trägers enthält, der ein Freisetzungsmittel zusammen mit (bezogen auf das Gesamtgewicht) 70 bis 30% hydratisierter Alkylhydroxycellulose, in welcher die Alkylgruppen bis zu 4 Kohlenstoffatome enthalten und mindestens eine der Alkylgruppen Propyl oder Butyl ist, und (bezogen auf das Gesamtgewicht) 30 bis 70% eines pharmazeutisch annehmbaren Polyalkylenoxids umfaßt, wobei der Hydratationsgrad der Alkylhydroxycellulose 5 bis 25 Gew.-% bezogen auf deren Gesamtegewicht, beträgt.

22. Verwendung nach Anspruch 21, worin die hydratisierte Alkylhydroxycellulose Propylhydroxymethylcellulose ist.

**Revendications**

1. Utilisation d'un composé représenté par la formule générale I:

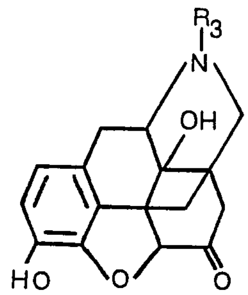

(I)

dans laquelle $R_3$ est du 3-méthyl-2-buténуl, de l'allyle, du cyclopropylméthyle ou du cyclobutylméthyle; et les sels pharmaceutiquement acceptables de celui-ci pour la fabrication de médicaments pour restaurer la motilité intestinale chez un patient souffrant d'une dysmotilité intestinale qui est indépendante de l'administration antérieure d'agonistes opiacés.

2. Utilisation selon la revendication 1, dans laquelle le médicament est fabriqué sous une forme appropriée pour l'administration orale.

3. Utilisation selon la revendication 1, dans laquelle le médicament est fabriqué sous une forme appropriée pour l'administration parentérale.

4. Utilisation selon la revendication 1, dans laquelle le médicament est fabriqué sous la forme d'une formule à libération prolongée.

5. Utilisation selon la revendication 4, dans laquelle ladite formule à libération prolongée est fabriquée pour contenir, en se basant sur le poids total, de 5 à 20% de composé(s) de formule I, incluant ses ou leurs sels pharmaceutiquement acceptables, et de 80 à 95% d'un support, ledit support comprenant un agent de libération en même temps que, en se basant sur le poids total, de 70% à 30% de cellulose hydroxyalkylée hydratée dans laquelle les groupes alkylés contiennent jusqu'à quatre atomes de carbone et au moins l'un d'eux est du propyle ou du butyle, et, en se basant sur le poids total, de 30% à 70% d'un oxyde de polyalkylène pharmaceutiquement acceptable, le degré d'hyratation de la cellulose hydroxyalkylée étant de 5% à 25% en poids, en se basant sur son poids total.

6. Utilisation selon l'une quelconque des revendications 1 à 5, dans laquelle le composé de la formule I est la naloxone ou la naltrexone.

7. Utilisation selon l'une quelconque des revendications 5 et 6, dans laquelle la celulose hydroxyalkylée hydratée est le cellulose hydroxyméthylée propylée.

8. Utilisation selon l'une quelconque des revendications 1 à 3 et 6, dans laquelle le médicament est destiné au traitement du syndrome de l'intestin irritable et/ou de la constipation.

9. Utilisation d'un composé représenté par la formule générale II:

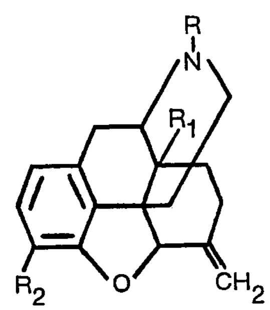

(II)

dans laquelle:

R est de l'allyle ou du cyclopropylméthyle;

$R_1$ est de l'hydrogène ou un hydroxy;

$R_2$ est un hydroxy ou un méthoxy;

et les sels pharmaceutiquement acceptables de celui-ci pour la fabrication de médicaments pour restaurer la motilité intestinale chez un patient souffrant d'une dysmotilité intestinale qui est indépendante de l'administration antérieure d'agonistes opiacés.

10. Utilisation selon la revendication 9, dans laquelle le médicament est fabriqué sous une forme appropriée pour l'administration orale.

11. Utilisation selon la revendication 9, dans laquelle le médicament est fabriqué sous la forme d'une formule à libération prolongée.

12. Utilisation selon la revendication 11, dans laquelle ladite formule à libération prolongée est fabriquée pour contenir, en se basant sur le poids total, de 5 à 20% de composé(s) de formule II, incluant ses ou leurs sels pharmaceutiquement acceptables, et de 80 à 95% d'un support, ledit support comprenant un agent de libération en même temps que, en se basant sur le poids total, de 70% à 30% de cellulose hydroxyalkylée hydratée dans laquelle les groupes alkylés contiennent jusqu'à quatre atomes de carbone et au moins l'un d'eux est du propyle ou du butyle, et, en se basant sur le poids total, de 30% à 70% d'un oxyde de polyalkylène pharmaceutiquement acceptable, le degrée d'hydratation de la cellulose hydroxyalkylée étant de 5% à 25% en poids, en se basant sur son poids total.

13. Utilisation selon l'une quelconque des revendications 9 à 12, dans laquelle le composé de formule II est du namlefène.

14. Utilisation selon l'une quelconque des revendications 9, 10 et 13, dans laquelle le médicament est destiné au traitement du syndrome de l'intestin irritable et/ou de la constipation.

15. Utilisation selon la revendication 12, dans laquelle la cellulose hydroxyalkylée hydratée est la cellulose hydroxyméthylée propylée.

16. Utilisation d'un composé représenté par la formule générale III:

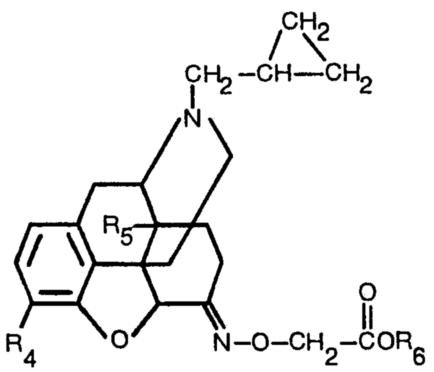

(III)

ou le compose déhydro- correspondant, dans laquelle:

$R_4$ est un méthoxy ou un hydroxy;

$R_5$ est de l'hydrogène ou un hydroxy;

$R_6$ est de l'hydrogène, du méthyle, de l'éthyle, du propyle, de l'allyle ou du benzyle;

et les sels pharmaceutiquement acceptables de celui-ci pour la fabrication de médicaments pour restaurer la motilité intestinale chez un patient souffrant d'une dysmotilité intestinale que est indépendante de l'administration antérieure d'agonistes opiacés.

17. Utilisation selon la revendication 16, dans laquelle le médicament est fabriqué sous une forme appropriée pour l'administration orale.

18. Utilisation selon la revendication 16, dans laquelle le médicament est fabriqué sous une forme appropriée pour l'administration parentérale.

19. Utilisation selon l'une quelconque des revendications 16 à 18, dans laquelle le médicament est destiné au traitement du syndrome de l'intestin irritable et/ou de la constipation.

20. Utilisation selon la revendication 16, dans laquelle le médicament est fabriqué sour la forme d'une formule à libération prolongée.

21. Utilisation selon la revendication 20, dans laquelle la formule à libération prolongée est fabriquée pour contenir, en se basant sur le poids total, de 5 à 20% de composé(s) de formule III, incluant le ou les composés déhydro- correspondants et les sels pharmaceutiquement acceptables, et de 80 à 95% d'un support, ledit support comprenant un agent de libération en même temps que, en se basant sur le poids total, de 70% à 30% de cellulose hydroxyalkylée hydratée dans laquelle les groupes alkylés contiennent jusqu'à quatre atomes de carbone et au moins l'un d'eux est du propyle ou du butyle, et, en se basant sur le poids total, de 30% à 70% d'un oxyde de polyalkylène pharmaceutiquement acceptable, le degré d'hydratation de la cellulose hydroxyalkylée étant de 5% à 25% en poids, en se basant sur son poids total.

22. Utilisation selon la revendication 21, dans laquelle la cellulose hydroxyalkylée hydratée est la cellulose hydroxyméthylée propylée.